(19) Europäisches Patentamt
European Patent Office
Office européen des brevets

(11) EP 1 545 441 B1

(12) # FASCICULE DE BREVET EUROPEEN

(45) Date de publication et mention de la délivrance du brevet:
**09.02.2011 Bulletin 2011/06**

(21) Numéro de dépôt: **03798229.5**

(22) Date de dépôt: **26.09.2003**

(51) Int Cl.:
***A61K 8/81*** (2006.01) ***A61K 8/90*** (2006.01)
***A61Q 1/04*** (2006.01) ***A61Q 1/06*** (2006.01)

(86) Numéro de dépôt international:
**PCT/FR2003/002843**

(87) Numéro de publication internationale:
**WO 2004/028489 (08.04.2004 Gazette 2004/15)**

(54) **ROUGE A LEVRES COMPRENANT UN POLYMERE SEQUENCE**

LIPPENSTIFT MIT EINEM SEQUENZIERTEM POLYMER

LIPSTICK COMPRISING A SEQUENCED POLYMER

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**20.12.2002 FR 0216437**
**21.05.2003 FR 0306121**

(43) Date de publication de la demande:
**29.06.2005 Bulletin 2005/26**

(73) Titulaire: **L'Oréal**
**75008 Paris (FR)**

(72) Inventeurs:
- **FERRARI, Véronique**
**F-94700 Maisons-Alfort (FR)**
- **BLIN, Xavier**
**F-75015 Paris (FR)**
- **LION, Bertrand**
**F-95270 Luzarches (FR)**

(74) Mandataire: **Kromer, Christophe**
**L'Oréal**
**D.I.P.I.**
**25-29 Quai Aulagnier**
**92600 Asnières (FR)**

(56) Documents cités:
**EP-A- 1 082 953**
**WO-A-00/28948**
**WO-A-98/42298**
**DE-A- 10 029 697**
**FR-A- 2 809 306**
**FR-A- 2 832 719**
**US-A- 6 153 206**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition cosmétique de maquillage des lèvres comprenant un polymère séquencé particulier.

Les compositions de rouge à lèvres sont couramment employées pour apporter une couleur esthétique aux lèvres. Ces produits de maquillage contiennent généralement des phases grasses telles que des cires et des huiles, des pigments et/ou charges et éventuellement des additifs comme des actifs cosmétiques ou dermatologiques.

Le brevet US 6 153 206 décrit des compositions cosmétiques et notamment des rouges à lèvres comprenant des copolymères éthyléniques linéaires préparés par GTP (Group Transfer Polymerisation). La demande de brevet FR 2 809 306 concerne l'utilisation dans des compositions cosmétiques de maquillage de copolymères éthyléniques séquences préparés par polymérisation radicalaire contrôlée. Enfin, la demande de brevet FR 2 832 719 non publiée à la date de dépôt de la demande concerne des copolymères éthyléniques séquences pouvant être préparés par polymérisation par voie anionique, par polymérisation radicalaire contrôlée ou par polymérisation radicalaire classique et leur utilisation en cosmétique pour l'amélioration du pouvoir coiffant et de la tenue.

Ces compositions, lorsqu'elles sont appliquées sur les lèvres, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une mauvaise tenue du film appliqué, nécessitant de renouveler régulièrement l'application de la composition de rouge à lèvres. Par ailleurs, le maquillage des lèvres peut aussi être altéré lors du contact avec des liquides, notamment de l'eau ou des boissons consommées par exemple lors d'un repas, ou bien encore des huiles comme les huiles alimentaires ou bien encore le sébum ou bien encore de la salive. tenue, en particulier de tenue après un contact avec de l'eau ou avec une huile alimentaire.

De façon plus précise, la présente invention a donc pour objet une composition cosmétique de maquillage des lèvres (ou rouge à lèvres) comprenant au moins un milieu liquide organique cosmétiquement acceptable et au moins un polymère éthylénique séquencé linéaire filmogène tel que décrit ci après, la composition étant notamment telle que définie ci-après.

Avantageusement, selon un premier mode de réalisation de la composition selon l'invention, le polymère éthylénique séquencé linéaire filmogène est exempt de styrène , ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence, la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité I supérieur ou égal à 2,8,
ledit polymère étant tel, que lorsqu'il est présent en quantité suffisante dans la composition, cette dernière est apte à former un dépôt ayant un indice de tenue supérieur ou égal à 80 %.

Avantageusement, selon un deuxième mode de réalisation de la composition selon l'invention, le polymère éthylénique séquencé linéaire filmogène est non élastomère, ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité I supérieur ou égal à 2,8,
ledit polymère étant tel, que lorsqu'il est présent en quantité suffisante dans la composition, cette dernière est apte à former un dépôt ayant un indice de tenue supérieur ou égal à 80 %.

L'invention a également pour objet un procédé cosmétique de maquillage des lèvres comprenant l'application sur les lèvres d'une composition telle que définie précédemment.

L'invention a également pour objet l'utilisation d'une composition telle que définie précédemment pour obtenir un dépôt de maquillage sur les lèvres ayant une bonne tenue, notamment une bonne tenue après un contact avec de

l'eau ou avec une huile alimentaire.

Le brevet US 6153206 décrit une composition cosmétique comprenant un polymère contenant des unités répétitives ayant une Tg allant de -10 à 75 °C et des unités répétitives ayant une Tg allant de 76 à 120 °C. Il décrit également des polymères blocs comprenant des blocs de poly méthyl méthacrylate et des blocs de polyisobutyl méthacrylate, et également des polymères triblocs comprenant des blocs de poly méthyl méthacrylate, des blocs de polyisobutyl méthacrylate et des blocs d'éthyl methacrylate.

Les documents FR-A-2809306, WO 03/046032 décrivent des compositions cosmétiques comprenant un polymère éthylénique séquencé. Dans les exemples, le seul polymère exemplifié à un indice de polydispersité respectivement égal à 2,2 et 1,16.

Avantageusement, le polymère éthylénique séquence linéaire filmogène utilisé dans la composition selon l'invention est tel que lorsqu'il est présent en quantité suffisante dans la composition, cette dernière est apte à former un dépôt ayant un indice de tenue supérieur ou égal à 80 %, de préférence supérieur ou égal à 85 %, de préférence inférieur ou égal à 90 %, de préférence supérieur ou égal à 95 %.

L'indice de tenue du dépôt obtenue avec la composition selon l'invention est déterminé selon le protocole de mesure décrit ci-après.

On prépare un support (rectangle de 40 mm X 70 mm) constitué d'un revêtement acrylique (adhésif acrylique hypoallergénique sur film polyéthylène vendu sous la dénomination BLENDERME ref FH5000-55113 par la société 3M Santé) collé sur une couche de mousse de polyéthylène adhésif sur la face opposée à celle sur laquelle est fixé le sparadrap (couche de mousse vendue sous la dénomination RE40X70EP3 de la société JOINT TECHNIQUE LYONNAIS IND).

On mesure à l'aide d'un colorimètre MINOLTA CR 300 la couleur $L^*_0a^*_0b^*_0$ du support , côté face revêtement acrylique.

On préchauffe le support ainsi préparé sur une plaque chauffante maintenue à la température de 40 °C pour que la surface du support soit maintenue à une température de 33 °C ± 1 °C.

Tout en laissant le support sur la plaque chauffante, on applique la composition sur toute la surface non adhésive du support (c'est-à-dire sur la surface du revêtement acrylique) en l'étalant à l'aide d'un pinceau pour obtenir un dépôt de la composition d'environ 15 μm puis on laisse sécher pendant 10 minutes.

Après séchage, on mesure la couleur L*a*b* du film ainsi obtenu.

On détermine alors la différence de couleur ΔE1 entre la couleur du film par rapport à la couleur du support nu par la relation suivante.

$$\Delta E1 = \sqrt{(L^*-L_o^*)^2 + (a^* - a_o^*)^2 + (b^* - b_o^*)^2}$$

Le support est ensuite collé par sa face adhésive (face adhésive de la couche de mousse) sur une enclume d'un diamètre de 20 mm et munie d'un pas de vis. Une éprouvette de l'ensemble support/dépôt est ensuite découpée à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Sur un papier blanc pour photocopieuse de grammage 80g/m2 , on dessine une bande de 33 mm de largeur et 29,7 cm de longueur, on trace un premier trait à 2 cm du bord de la feuille, puis un deuxième trait à 5 cm du bord de la feuille, les premier et deuxième traits délimitant ainsi une case sur la bande ; puis on dispose une première marque et une deuxième marque situées dans la bande respectivement aux repères 8 cm et 16 cm du deuxième trait. On place sur la première marque 20 μl d'eau et sur la deuxième marque 10 μl d'huile de tournesol raffinée (vendue par la société LESIEUR).

Le papier blanc est placé sur le socle de la presse puis on presse l'éprouvette placée sur la case de la bande de papier à une pression d'environ 300 g/cm$^2$ exercée pendant 30 secondes. Puis on relève la presse et on place à nouveau l'éprouvette juste après le deuxième trait (donc à côté de la case), on effectue à nouveau une pression d'environ 300 g/cm$^2$ et on déplace, de manière rectiligne dès le contact effectué, le papier avec une vitesse de 1 cm/s, sur toute la longueur de la bande de telle sorte que l'éprouvette traverse les dépôts d'eau et d'huile. Après retrait de l'éprouvette, une partie du dépôt a transféré sur le papier. On mesure alors la couleur L*', a*', b*' du dépôt resté sur l'éprouvette.

On détermine alors la différence de couleur ΔE2 entre la couleur du dépôt resté sur l'éprouvette par rapport à la couleur du support nu par la relation suivante.

$$\Delta E2 = \sqrt{(L^{*\prime}-L_o^{*})^2 + (a^{*\prime} - a_o^{*})^2 + (b^{*\prime} - b_o^{*})^2}$$

L'indice de tenue de la composition, exprimée en pourcentage, est égale au rapport :

$$100 \ X \ \Delta E2 / \Delta E1$$

La mesure est effectuée sur 6 supports à la suite et la valeur de transfert correspond à la moyenne des 6 mesures obtenues avec les 6 supports.

Par liquide organique, on entend tout produit non aqueux liquide à température ambiante (25 °C).

Par milieu liquide organique cosmétiquement acceptable, on entend un milieu comprenant au moins un composé organique liquide à température ambiante (25 °C) et pression atmosphérique ($10^5$ Pa) compatible avec les matières kératiniques, notamment la peau, les lèvres, telles que les huiles ou les solvants organiques couramment employés dans les compositions cosmétiques.

La composition selon l'invention peut comprendre un polymère séquencé éthylénique séquencé linéaire filmogène exempt de styrène.

Par "polymère exempt de styrène", on entend un polymère comprenant moins de 10%, de préférence moins de 5%, de préférence moins de 2%, de préférence encore moins de 1 % en poids, voire ne contient pas, de monomère styrénique tels que le styrène ou les dérivés du styrène comme par exemple le méthylstyrène, le chlorostyrène ou le chlorométhylstyrène.

Selon un mode de réalisation, le polymère séquencé de la composition selon l'invention est issu de monomères éthyléniques aliphatiques. Par monomère aliphatique, on entend un monomère ne comprenant aucun groupe aromatique.

Par polymère "éthylénique" , on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes.

Le polymère est un polymère à structure linéaire. Par opposition, un polymère a structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

De façon préférentielle, le polymère utilisé dans la composition selon l'invention ne comprend pas d'atomes de silicium dans son squelette. Par "squelette", on entend la chaîne principale du polymère, par opposition aux chaînes latérales pendantes.

Avantageusement, le polymère séquencé comprend au moins une première séquence et au moins une deuxième séquence telles que décrites précédemment.

Par "au moins" une séquence, on entend une ou plusieurs séquences.

On précise que dans ce qui précède et ce qui suit les termes "première" et "deuxième" séquences ne conditionnent nullement l'ordre desdites séquences (ou blocs) dans la structure du polymère.

De préférence, les première et deuxième séquences dudit polymère séquencé sont incompatibles l'une avec l'autre.

Par "séquences incompatibles l'une avec l'autre", on entend que le mélange formé du polymère correspondant à la première séquence et du polymère correspondant à la deuxième séquence, n'est pas miscible dans le liquide organique majoritaire en poids du milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que

ii) chacun des polymères correspondant au première et seconde séquences a une masse moléculaire moyenne (en poids ou en nombre) égale à celle du polymère séquencé +/- 15%.

Dans le cas où le milieu liquide organique comprend un mélange de liquides organiques, et dans l'hypothèse de deux ou plusieurs liquides organiques présents en proportions massiques identiques, ledit mélange de polymères est non miscible dans au moins l'un d'entre eux.

Bien entendu, dans le cas où le milieu liquide organique comprend un unique liquide organique, ce dernier est le liquide organique majoritaire.

Avantageusement, le liquide organique majoritaire de la composition est le solvant organique de polymérisation du polymère séquencé ou le solvant organique majoritaire du mélanges de solvant organiques de polymérisation du polymère séquencé.

De préférence, le polymère séquencé utilisé dans la composition selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, le polymère séquencé utilisé dans la composition selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50 %.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($I_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$).

La recouvrance retardée en % ($R_{2h}$) est donnée par la formule ci-après:

$$R_{2h}= (\varepsilon_{max} - \varepsilon_{2h})/ \varepsilon_{max}) \times 100$$

A titre purement indicatif, un polymère utilisé selon un mode de réalisation de l'invention possède une recouvrance instantanée $R_i$ de 10% et une recouvrance retardée $R_{2h}$ de 30%.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère utilisé dans les compositions selon l'invention est de préférence inférieure ou égale à 300 000, elle va par exemples de 35 000 à 200 000, et mieux de 45 000 à 150 000.

La masse moyenne en nombre (Mn) du polymère utilisé dans les compositions selon l'invention est de préférence inférieure ou égale à 70 000, elle va par exemple de 10 000 à 60 000, et mieux de 12 000 à 50 000.

L'indice de polydispersité du polymère séquencé utilisé dans les compositions selon l'invention va notamment de 2,8 à 6.

Chaque séquence ou bloc du polymère utilisé dans les compositions selon l'invention est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

De préférence, la séquence intermédiaire est issue essentiellement de monomères constitutifs de la première séquence et de la deuxième séquence. Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) ,$$

$\overline{\omega}_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

Avantageusement, les première et deuxième séquences du polymère sont telles que l'écart entre les températures de transition vitreuse des première et deuxième séquences est généralement supérieur à 10°C, de préférence supérieur à 20°C, et mieux supérieur à 30°C.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont incluses.

a) Séquence ayant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.

La séquence ayant une Tg supérieure ou égale à 40°C peut être un homopolymère ou un copolymère.

Dans le cas où cette séquence est un homopolymère, elle est issue de monomère dont l'homopolymère a une température de transition vitreuse supérieure ou égale à 40°C.

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères dont l'homopolymère a une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères dont l'homopolymère a une Tg inférieures à 40°C, choisis parmi les monomères dont l'homopolymère a une Tg comprise entre 20 à 40°C et/ou les monomères dont l'homopolymère a une Tg inférieure ou égale à 20°C, par exemple une Tg allant de -100 à 20°C, de préférence inférieure à 15°C, notamment allant de - 80°C à 15°C et mieux inférieure à 10°C, par exemple allant de -50°C à 0°C, tels que décrits plus loin, .

Les monomères dont l'homopolymère a une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle
  ou isobutyle ou $R_1$ représente un groupe cycloalkyle $C_4$ à $C_{12}$,

- les acrylates de formule $CH_2 = CH\text{-}COOR_2$
  dans laquelle $R_2$ représente un groupe cycloalkyle en $C_4$ à $C_{12}$ tel que un groupe isobornyle ou un groupe tertio butyle,

- les (méth)acrylamides de formule :

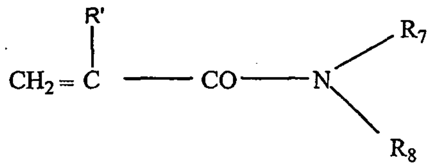

  où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
  et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide,

- et leurs mélanges.

Des monomères principaux particulièrement préférés sont le méthacrylate de méthyle, le (méth)acrylate d'isobutyle, le (méth)acrylate d'isobornyle et leurs mélange.

b) Séquence avant une Tg inférieure ou égale à 20°C

La séquence ayant une Tg inférieure ou égale à 20°C a par exemple une Tg allant de -100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.

Elle peut par exemple comprendre

- un ou plusieurs monomères dont l'homopolymère correspondant a une Tg inférieure ou égale à 20°C, par exemple une Tg allant de -100°C à 20°C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-}CO\text{-}O\text{-}CH = CH_2$
  où $R_5$ représente un groupe alkyle en $C_4$ à $C_{12}$ linéaire ou ramifié ;

  - les éthers d'alcool vinylique et d'alcool en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

<u>c) Séquence ayant une Tg comprise entre 20 et 40°C</u>

La séquence qui a une Tg comprise entre 20 et 40°C peut être un homopolymère ou un copolymère.

Dans le cas où cette séquence est un homopolymère, elle est issue de monomère (ou monomère principal) dont l'homopolymère a une température de transition vitreuse comprises entre 20 et 40°C.

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.

Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,

lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

De préférence, le polymère utilisé dans les compositions selon l'invention est exempt de styrène.

Chacune des séquences peut néanmoins contenir en proportion minoritaire au moins un monomère constitutif de l'autre séquence.

Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux

cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)-COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)-COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris (triméthylsiloxy) silane,

- et leurs mélanges.

Des monomères additionnels particulièrement préférés sont l'acide acrylique, l'acide méthacrylique, le méthacrylate de trifluoroéthyle et leurs mélanges.

Selon un mode préféré de réalisation, le polymère utilisé dans les compositions selon l'invention est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

De préférence, chacune des première et deuxième séquences comprend au moins un monomère choisi parmi les esters d'acide (méth)acrylique tels que définis précédemment, et éventuellement un monomère choisi parmi l'acide (méth)acrylique, et leurs mélanges.

Avantageusement, chacune des première et deuxième séquences est issue en totalité d'au moins un monomère choisi parmi les esters d'acide (méth)acrylique tels que définis précédemment, et éventuellement un monomère choisi parmi l'acide (méth)acrylique, et leurs mélanges.

Le polymère utilisé dans les compositions selon l'invention peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,

- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.
Selon un premier mode de réalisation, le polymère utilisé dans les compositions selon l'invention comprend au moins une (notamment une) première séquence ayant une Tg supérieure ou égale à 40°C, telle que décrite plus haut au a) et au moins une (notamment une) deuxième séquence ayant une Tg inférieure ou égale à 20°C, telle que décrite plus haut au b).
De préférence, la première séquence ayant une Tg supérieure ou égale à 40°C est un copolymère issu de monomères dont l'homopolymère a une température de transition vitreuse supérieure ou égale à 40°C, tels que les monomère décrits précédemment. Avantageusement, la deuxième séquence ayant une Tg inférieure ou égale à 20°C est un homopolymère, notamment issu de monomère tels que décrits précédemment.
De préférence, la proportion de la séquence ayant une Tg supérieure ou égale à 40°C va de 20 à 90% en poids du polymère, mieux de 30 à 80% et encore mieux de 50 à 70%.
De préférence, la proportion de la séquence ayant une Tg inférieure ou égale à 20°C va de 5 à 75% en poids du polymère, de préférence de 15 à 50% et mieux de 25 à 45%.
Ainsi, selon une première variante, le polymère utilisé dans les compositions selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante le polymère utilisé dans les compositions selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère utilisé dans les compositions selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de
- 85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère utilisé dans les compositions selon l'invention peut comprendre :

- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère utilisé dans les compositions selon l'invention peut comprendre :

- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère utilisé dans les compositions selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/ acrylate d'isobutyle.

Selon une septième variante, le polymère utilisé dans les compositions selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobornyle/ acrylate d'isobutyle.

Selon une huitième variante, le polymère selon l'invention peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Selon un second mode de réalisation, le polymère utilisé dans les compositions selon l'invention comprend au moins une (notamment une) première séquence ayant une température de transition vitreuse (Tg) comprise entre 20 et 40°C, conforme aux séquences décrites au c) et au moins une (notamment une) deuxième séquence ayant une température de transition vitreuse inférieure ou égale à 20°C, telle que décrite plus haut au b) ou une température de transition vitreuse supérieure ou égale à 40°C, telle que décrite au a) ci-dessus.
De préférence, la proportion de la première séquence ayant une Tg comprise entre 20 et 40°C va de 10 à 85% en poids du polymère, mieux de 30 à 80% et encore mieux de 50 à 70%.
Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.
Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.
De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C. .Avantageusement, la deuxième séquence ayant une Tg inférieure ou égale à 20°C ou ayant une Tg supérieure ou égale à 40°C est un homopolymère.
Ainsi, selon première variante de ce second mode de réalisation, le polymère utilisé dans les compositions selon l'invention peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et

- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère utilisé dans les compositions selon l'invention peut comprendre :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère utilisé dans les compositions selon l'invention peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg isupérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

Le polymère séquencé peut être présent dans la composition selon l'invention en une teneur allant de 0,1 % à 60 % en poids, par rapport au poids total de la composition, de préférence allant de 0,5 % à 50 % en poids, et de préférence encore allant de 1 % à 40 % en poids.

Selon un mode de réalisation particulièrement préféré, le milieu liquide organique de la composition contient au moins un liquide organique qui est le ou un des solvants organique(s) de polymérisation du polymère séquencé tel que décrit précédemment. Avantageusement, ledit solvant organique de polymérisation est le liquide organique majoritaire en poids présent dans le milieu liquide organique de la composition cosmétique.

La composition cosmétique selon l'invention comprend un milieu cosmétiquement acceptable, c'est-à-dire un milieu compatible avec les matières kératiniques, comme la peau, les lèvres, les cheveux, les cils, les sourcils et les ongles.

La composition selon l'invention peut comprendre au moins une huile volatile.

Par "huile", on entend tout milieu non aqueux liquide à température ambiante (25°C) et pression atmosphérique (760mm de Hg), compatible avec une application sur la peau, les muqueuses (lèvres) et/ou les phanères (ongles, cils, sourcils, cheveux).

Par huile volatile, on entend tout milieu non aqueux susceptible de s'évaporer de la peau ou des lèvres, en moins d'une heure, ayant notamment une pression de vapeur, à température ambiante et pression atmosphérique allant de $10^{-3}$ à 300 mm de Hg (0,13 Pa à 40 000 Pa).

Selon l'invention, on peut utiliser une ou plusieurs huiles volatiles.

Ces huiles peuvent être des huiles hydrocarbonées ou des huiles siliconées comportant éventuellement des groupements alkyle ou alkoxy pendants ou en bout de chaîne siliconée.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éthers, acide carboxylique, amine et/ou amide.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltri-siloxane, l'heptaméthyloctyltrisiloxane, l'octaméthyltrisiloxane, le décaméthyltétrasiloxane et leurs mélanges.

Comme autre huile volatile utilisable dans l'invention, on préfère notamment les isoparaffines en $C_8$-$C_{16}$ comme l'isododécane, l'isodécane, l'isohexadécane et par exemple les huiles vendues sous les noms commerciaux d'ISOPAR, de PERMETHYL et notamment l'isododécane (PERMETHYL 99 A).

L'huile volatile peut être présente dans la composition selon l'invention en une teneur allant de 1 % à 70 % en poids, par rapport au poids total de la composition, de préférence allant de 5 % à 50 % en poids, et préférentiellement allant de 10 % à 35 % en poids.

La composition selon l'invention peut comprendre une huile non volatile.

On entend par huile non volatile une huile susceptible de rester sur la peau à température ambiante (25 °C) et pression atmosphérique au moins une heure et ayant notamment une pression de vapeur à température ambiante (25 °C) et pression atmosphérique, non nulle, inférieure à 0,01 mm de Hg (1,33 Pa).

L'huile non volatile peut être choisie parmi les huiles non volatiles siliconées ou hydrocarbonées.

L'huile non volatile peut être choisie parmi les huiles non volatiles polaires ou apolaires, les huiles non volatiles polaires, et leurs mélanges.

L'huile non volatile peut être présente dans la composition selon l'invention en une teneur allant de 1 % à 80 % en poids, par rapport au poids total de la composition, de préférence allant de 5 % à 60 % en poids, et préférentiellement allant de 10 % à 50 % en poids, notamment allant de 20 % à 50 % en poids.

Comme huile non volatile utilisable dans l'invention, on peut citer :

- les huiles non volatile hydrocarbonées telles que l'huile de paraffine ( ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en $C_{12}$-$C_{36}$, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine ; les acides gras supérieurs, notamment en $C_{14}$-$C_{22}$, tels que l'acide myristique, l'acide palmitique, l'acide stéarique, l'acide béhénique, l'acide oléique, l'acide linoléique, l'acide linolénique ou l'acide isostéarique ; les alcools gras supérieurs, notamment en $C_{16}$-$C_{22}$, tels que le cétanol, l'alcool oléique, l'alcool linoléique ou linolénique, l'alcool isostéarique ou l'octyl dodécanol ; et leurs mélanges.
- les huiles siliconées non volatiles telles que les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes ; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ; et leurs mélanges

La composition selon l'invention peut également comprendre au moins corps gras solides à température ambiante notamment choisi parmi les cires, les corps gras pâteux, les gommes et leurs mélanges. Ces corps gras peuvent être d'origine animale, végétale, minérale ou synthétique.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50 % en poids de cires, par rapport au poids total de la composition et mieux de 1 à 30 % en poids.

La composition peut ainsi comprendre de l'eau ou un mélange d'eau et de solvant(s) organique(s) hydrophile (s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition peut comprendre, outre le polymère séquencé décrit précédemment selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), sous forme de crème, de stick, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte ou d'un stick anhydre. La composition peut être une composition non rinçée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'une quelconque des revendications qui précèdent.

Le récipient peut être sous toute forme adéquate. Il peut être notamment sous forme d'un flacon, d'un tube, d'un étui, d'une boite, ou d'un boîtier.

L'élément de fermeture peut être sous forme d'un bouchon amovible, d'un couvercle, ou d'une capsule, notamment du type comportant un corps fixé au récipient et une casquette articulée sur le corps. Il peut être également sous forme d'un élément assurant la fermeture sélective du récipient, notamment un clapit.

Le récipient peut être associé à un applicateur. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante).

Le contenu des brevets ou demandes de brevets cités précédemment sont incorporés par référence dans la

présente demande.

L'invention est illustrée plus en détails par les exemples décrits ci-après.

**Exemple 1 :**

**Préparation d'un polymère de poly(acrylate d'isobornyle/méthacrylate d'isobutyle/acrylate d'éthyl-2 hexyle)**

100 g d'isododécane sont introduits dans un réacteur de 1 litre, puis on augmente la température de façon à passer de la température ambiante (25°C) à 90°C en 1 heure.

On ajoute ensuite, à 90°C et en 1 heure, 120 g d'acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobutyle) ayant une Tg de 80°C, une deuxième séquence polyacrylate d'éthyl-2 hexyle ayant une Tg de - 70°C et une séquence intermédiaire qui est un polymère statistique acrylate d'isobornyle/méthacrylate d'isobutyle/acrylate d'éthyl-2 hexyle.

Ce polymère présente une masse moyenne en poids de 77 000 et une masse moyenne en nombre de 19 000, soit un indice de polydispersité I de 4,05.

**Exemple 2 :**

**Préparation d'un Polymère de poly(acrylate d'isobornyle/méthacrylate d'isobornyle lacrylate d'éthyl-2 hexyle)**

100 g d'isododécane sont introduits dans un réacteur de 1 litre, puis on augmente la température de façon à passer de la température ambiante (25°C) à 90°C en 1 heure.

On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité I de 4.89.

**Exemples 3 et 4 :**

On a préparé un stick de rouge à lèvres ayant la composition suivante :

| | |
|---|---|
| Polymère de l'exemple 1 ou 2 à 50 % en poids dans l'isododécane | 65 g |
| Octyldodécanol | 11,34 g |
| Cire de polyéthylène (Performalene 500 polyethylène de New phase Technologies) | 13 g |
| Mélange d'alcools gras en C30-C50 et d'hydrocarbure en C30-C50 (80/20) (Performacol 550 de New Phase Technologies) | 2 g |
| Pigments | 8,66 g |

On réalise un broyat pigmentaire des pigments dans l'octyldodécanol en effectuant 3 passages du mélange à

la broyeuse tri cylindres.

On fond le mélange de cires et de broyat pigmentaire à 100 °C sous agitation Rayneri et l'on maintient l'agitation pendant 40 minutes. On abaisse ensuite la température du mélange à 80 °C et on ajoute le polymère mélangé à l'isododécane. On laisse sous agitation pendant 15 minutes puis le mélange est coulé dans un moule chauffé à 42 °C puis refroidi dans un congélateur à - 18 °C pendant 30 minutes.

Les bâtons obtenus sont conditionnés dans un article de conditionnement.

Le rouge à lèvres permet d'obtenir un maquillage présentant de bonne propriétés de non transfert et de tenue.

On mesuré l'indice de tenue du rouge à lèvres obtenu selon le protocole de mesure décrit précédemment.

Les rouges à lèvres des exemples 3 et 4 forment respectivement un film ayant un indice de tenue 100 %.

__Exemple 5:__

On a préparé un stick de rouge à lèvres ayant la composition suivante :

| | |
|---|---|
| Polymère de l'exemple 1 à 50 % en poids dans l'isododécane | 50 g |
| Octyldodécanol | 11,34 g |
| Cire de polyéthylène (Performalene 500 polyethylène de New phase Technologies) | 13 g |
| Mélange d'alcools gras en C30-C50 et d'hydrocarbure en C30-C50 (80/20) (Performacol 550 de New Phase Technologies) | 2 g |
| Pigments | 8,66 g |
| Isododécane | 15 g |

Ce rouge à lèvres forme un film ayant un indice de tenue égal à 98,6 % , mesuré selon le protocole décrit précédemment.

__Exemple 6 :__

On a préparé un stick de rouge à lèvres ayant la composition suivante :

| | |
|---|---|
| Polymère de l'exemple 1 à 50 % en poids dans l'isododécane | 30 g |
| Octyldodécanol | 11,34 g |
| Cire de polyéthylène (Performalene 500 polyethylène de New phase Technologies) | 15g |
| Mélange d'alcools gras en C30-C50 et d'hydrocarbure en C30-C50 (80/20) (Performacol 550 de New Phase Technologies) | 2 g |
| Pigments | 8,66 g |
| Isododécane | 35 g |

Ce rouge à lèvres forme un film ayant un indice de tenue égal à 98,6 % , mesuré selon le protocole décrit précédemment.

## Revendications

**1.** Composition de maquillage des lèvres comprenant au moins un milieu liquide organique cosmétiquement acceptable et au moins un polymère éthylénique séquencé linéaire filmogène exempt de styrène,
ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ; et ledit polymère séquencé ayant un indice de polydispersité I supérieur ou égal à 2,8, ledit polymère étant tel, que lorsqu'il est présent en quantité suffisante dans la composition, cette dernière est apte à former un dépôt ayant un indice de tenue supérieur ou égal à 80 %, déterminé selon le protocole de mesure décrit dans la description.

2. Composition de maquillage des lèvres comprenant au moins un milieu liquide organique cosmétiquement acceptable et au moins un polymère éthylénique séquencé linéaire filmogène non élastomère, ledit polymère séquencé contenant des première et deuxième séquences reliées entre elles par un segment intermédiaire qui est un polymère statistique comprenant au moins un monomère constitutif de la première séquence et au moins un monomère constitutif de la deuxième séquence, la première séquence du polymère étant choisie parmi :

   - a) une séquence ayant une Tg supérieure ou égale à 40°C,
   - b) une séquence ayant une Tg inférieure ou égale à 20°C,
   - c) une séquence ayant une Tg comprise entre 20 et 40°C, et

   la deuxième séquence étant choisie dans une catégorie a), b) ou c) différente de la première séquence ; et ledit polymère séquencé ayant un indice de polydispersité I supérieur ou égal à 2,8, ledit polymère étant tel, que lorsqu'il est présent en quantité suffisante dans la composition, cette dernière est apte à former un dépôt ayant un indice de tenue supérieur ou égal à 80 %, déterminé selon le protocole de mesure décrit dans la description

3. Composition selon la revendication 1 ou 2, **caractérisée par le fait qu'**elle est apte à former un dépôt ayant un indice de tenue supérieur ou égal à 85 %, de préférence inférieur ou égal à 90 %, de préférence supérieur ou égal à 95 %.

4. Composition selon l'une des revendications précédentes, **caractérisée par le fait que** le polymère séquencé n'est pas soluble à une teneur en matière active d'au moins 1% en poids dans l'eau ou dans un mélange d'eau et de monoalcools inférieurs linéaires ou ramifiés ayant de 2 à 5 atomes de carbone, sans modification de pH, à température ambiante (25°C).

5. Composition selon l'une des revendications précédentes, **caractérisée par le fait que** la séquence intermédiaire a une température de transition vitreuse comprise entre les températures de transition vitreuse des première et deuxième séquences.

6. Composition selon l'une quelconque des revendications précédentes, **caractérisée par le fait que** les première et deuxième séquences dudit polymère séquencé sont incompatibles l'une avec l'autre.

7. Composition selon l'une des revendications précédentes, **caractérisée par le fait que** le polymère séquencé a un indice de polydispersité compris entre 2,8 et 6.

8. Composition selon l'une quelconque des revendications précédentes, **caractérisée par le fait que** le polymère séquencé comprend au moins une première séquence ayant une température de transition vitreuse (Tg) supérieure ou égale à 40°C et au moins une deuxième séquence ayant une température de transition vitreuse inférieure ou égale à 20°C.

9. Composition selon la revendication précédente, **caractérisée par le fait que** la proportion de la première séquence va de 20 à 90% en poids du polymère, mieux de 30 à 80% et encore mieux de 50 à 70%.

10. Composition selon la revendication 8 ou 9, **caractérisée par le fait que** la proportion de la deuxième séquence ayant une Tg inférieure ou égale à 20°C va de 5 à 75% en poids du polymère, mieux de 15 à 50% et encore mieux de 25 à 45%.

11. Composition selon l'une des revendications 1 à 7, **caractérisée par le fait que** le polymère séquencé comprend au moins une première séquence ayant une température de transition vitreuse (Tg) comprise entre 20 et 40°C et au moins une deuxième séquence ayant une température de transition vitreuse inférieure ou égale à 20°C ou une température de transition vitreuse supérieure ou égale à 40°C.

**12.** Composition selon la revendication précédente, **caractérisée par le fait que** la proportion de la première séquence ayant une Tg comprise entre 20 et 40°C va de 10 à 85% en poids du polymère, mieux de 30 à 80% et encore mieux de 50 à 70%.

**13.** Composition selon la revendication 11 ou 12, **caractérisée par le fait que** la deuxième séquence a une Tg supérieure ou égale à 40 °C.

**14.** Composition selon l'une quelconque des revendications 11 à 13, **caractérisée par le fait que** la proportion de la deuxième séquence ayant une Tg supérieure ou égale à 40°C va de 10 à 85%, de préférence de 20 à 70% et mieux de 30 à 70% en poids du polymère.

**15.** Composition selon la revendication 11 ou 12, **caractérisée par le fait que** la deuxième séquence a une Tg inférieure ou égale à 20 °C.

**16.** Composition selon l'une des revendications 7 à 9 et 14, 15, **caractérisé par le fait que** la proportion de la séquence ayant une température de transition vitreuse inférieure ou égale à 20°C va de 20 à 90% en poids du polymère, mieux de 30 à 80% et encore mieux de 50 à 70%.

**17.** Composition selon l'une quelconque des revendications précédentes, **caractérisée par le fait que** la séquence ayant une Tg supérieure ou égale à 40°C est issue en totalité ou en partie de un ou plusieurs monomères dont l'homopolymère a une température de transition vitreuse supérieure ou égale à 40°C, notamment une Tg allant de 40 à 150 °C, de préférence supérieure ou égale à 50°C, notamment allant de 50°C à 120°C, et préférentiellement supérieure ou égale à 60°C, notamment allant de 60°C à 120°C.

**18.** Composition selon la revendication précédente, **caractérisée par le fait que** la séquence ayant une Tg supérieure ou égale à 40 °C est un copolymère issu de monomères dont l'homopolymère a une température de transition vitreuse supérieure ou égale à 40°C.

**19.** Composition selon l'une des revendications 17 ou 18, **caractérisé par le fait que** les monomères dont l'homopolymère a une température de transition vitreuse supérieure ou égale à 40°C sont choisis parmi les monomères suivants :

- les méthacrylates de formule $CH_2 = C(CH_3)\text{-}COOR_1$
dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

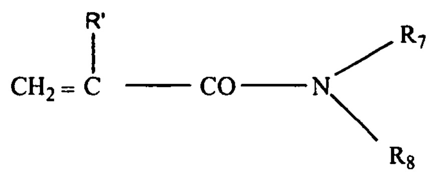

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.
- et leurs mélanges.

**20.** Composition selon l'une quelconque des revendications 17 à 19, **caractérisée par le fait que** les monomères dont l'homopolymère a une température de transition vitreuse supérieure ou égale à 40°C sont choisis parmi le méthacrylate de méthyle, le méthacrylate d'isobutyle, le (méth)acrylate d'isobornyle et leurs mélanges.

**21.** Composition selon l'une quelconque des revendications 1 à 17 et 19, 20, **caractérisée par le fait que** la séquence ayant une Tg supérieure ou égale à 40°C est un homopolymère.

**22.** Composition selon l'une quelconque des revendications 1 à 11 et 15, 16, **caractérisée par le fait que** la séquence ayant une Tg inférieure ou égale à 20°C est issue en totalité ou en partie de un ou plusieurs monomères dont l'homopolymère a une température de transition vitreuse inférieure ou égale à 20°C, notamment allant de -100 à 20°C, de préférence inférieure ou égale à 15°C, notamment allant de -80°C à 15°C et préférentiellement inférieure ou égale à 10°C, notamment allant de -50°C à 0°C.

**23.** Composition selon la revendication précédente, **caractérisée par le fait que** les monomères dont l'homopolymère a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$
où $R_5$ représente un groupe alkyle en $C_4$ à $C_{12}$ linéaire ou ramifié ;
- les éthers de vinyle et d'alkyle en $C_4$ à $C_{12}$, tels que l'éther de vinyle et de méthyle et l'éther de vinyle et d'éthyl ;
- les N-alkyl en $C_4$ à $C_{12}$ acrylamides, tels que le N-octylacrylamide,
et leurs mélanges.

**24.** Composition selon la revendication 22 ou 23, **caractérisée par le fait que** les monomères dont l'homopolymère a une température de transition vitreuse inférieure ou égale à 20°C sont choisis parmi les acrylates d'alkyle dont la chaîne alkyle comprend de 1 à 10 atomes de carbone, à l'exception du groupe tertiobutyle

**25.** Composition selon l'une quelconque des revendications 1 à 12 et 16 à 24, **caractérisée par le fait que** la séquence ayant une température de transition vitreuse inférieure ou égale à 20°C est un homopolymère.

**26.** Composition selon l'une quelconque des revendications 1 à 6 et 11 à 25, **caractérisée par le fait que** la séquence ayant une Tg comprise entre 20 et 40°C est issue en totalité ou en partie de un ou de plusieurs monomères dont l'homopolymère a une température de transition vitreuse comprise entre 20 et 40°C.

**27.** Composition selon l'une quelconque des revendications 1 à 6 et 11 à 26, **caractérisée par le fait que** la séquence ayant une Tg comprise entre 20 et 40 °C est un homopolymère d'un monomère choisi parmi le méthacrylate de n-butyle, l'acrylate de cyclodécyle, l'acrylate de néopentyle, l'isodécylacrylamide.

**28.** Composition selon l'une quelconque des revendications 1 à 6 et 11 à 26, **caractérisée par le fait que** la séquence ayant une Tg comprise entre 20 et 40°C est un copolymère issu en totalité ou en partie de :

- monomères dont l'homopolymère a une Tg supérieure ou égale à 40°C, notamment une Tg allant de 40°C à 150°C, de préférence supérieure ou égale à 50°C, notamment allant de 50 à 120°C, et préférentiellement supérieure ou égale à 60°C, notamment allant de 60°C à 120°C,
- et de monomères dont l'homopolymère a une Tg inférieure ou égale à 20°C, notamment allant de -100 à 20°C, de préférence inférieure ou égale à 15°C, notamment allant de -80°C à 15°C, et préférentiellement inférieure ou égale à 10°C, par exemple allant de -50°C à 0°C.

**29.** Composition selon l'une quelconque des revendications 1 à 6 et 11 à 26 et 28, **caractérisée par le fait que** la séquence ayant une Tg comprise entre 20 et 40°C est issue en totalité ou en partie de monomères choisis parmi le méthacrylate de méthyle, le (méth)acrylate d'isobornyle, le méthacrylate de trifuoroéthyle, l'acrylate de butyle, l'acrylate d'éthyl-2 hexyle et leurs mélanges.

**30.** Composition selon l'une quelconque des revendications 1 à 20 et 22 à 24, et 26, 28, 29, **caractérisée par le fait que** la première séquence et/ou la deuxième séquence comprend au moins un monomère additionnel.

**31.** Composition selon la revendication précédente, **caractérisée par le fait que** le monomère additionnel est choisi

parmi les monomères hydrophiles, les monomères à insaturation éthylénique comprenant un ou plusieurs atomes de silicium et leurs mélanges.

**32.** Composition selon la revendication 30 ou 31, **caractérisée par le fait que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_6$
dans laquelle $R_6$ représente un groupe alkyle linéaire ou ramifié, contenant de 1 à 4 atomes de carbone, ledit groupe alkyle étant substitué par un ou plusieurs substituants choisis parmi le groupe hydroxyle et les atomes d'halogènes,
- les méthacrylates de formule $CH_2 = C(CH_3)\text{-}COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi le groupe hydroxyle et les atomes d'halogènes ;
- les acrylates de formule $CH_2 = CHCOOR_{10}$,
$R_{10}$ représentant un groupe alkyle en $C_1$ à $C_{12}$ linéaire ou ramifié substitué par un ou plusieurs substituants choisis parmi le groupe hydroxyle et les atomes d'halogène, ou $R_{10}$ représente un alkyle($C_1$-$C_{12}$)-O-POE (polyoxyéthylène) avec répétition du motif oxyéthylène de 5 à 30 fois, ou $R_{10}$ représente un groupement polyoxyéthylèné comprenant de 5 à 30 motifs d'oxyde d'éthylène
- les monomères à insaturation(s) éthylénique(s) comprenant au moins une fonction amine tertiaire,
et leurs mélanges.

**33.** Composition selon l'une quelconque des revendications 30 à 32, **caractérisée par le fait que** le ou les monomères additionnels sont choisis parmi l'acide acrylique, l'acide méthacrylique, le méthacrylate de trifluoroéthyle et leurs mélanges.

**34.** Composition selon l'une des revendications 30 à 33, **caractérisée par le fait que** le ou les monomères additionnel (s) représente(nt) de 1 à 30% en poids du' poids total des première et/ou deuxième séquences.

**35.** Composition selon l'une des revendications 1 à 33, **caractérisée par le fait que** chacune des première et deuxième séquence comprend au moins un monomère choisi parmi les esters d'acide (méth)acrylique, et éventuellement au moins un monomère choisi parmi l'acide (méth)acrylique, et leurs mélanges.

**36.** Composition selon l'une des revendications 1 à 35, **caractérisée par le fait que** chacune des première et deuxième séquence est issue en totalité d'au moins un monomère choisi parmi les esters d'acide (méth)acrylique, et éventuellement d'au moins un monomère choisi parmi l'acide (méth)acrylique, et leurs mélanges

**37.** Composition selon l'une quelconque des revendications 2 à 38, **caractérisée par le fait que** le polymère séquencé est exempt de styrène.

**38.** Composition selon l'une des revendications précédentes, **caractérisée par le fait que** le polymère séquencé a une masse moyenne en poids (Mw) inférieure ou égale à 300 000, de préférence allant de 35 000 à 200 000, et mieux allant de 45 000 à 150 000.

**39.** Composition selon l'une des revendications précédentes, **caractérisée par le fait que** le polymère séquencé a masse moyenne en nombre (Mn) est inférieure ou égale à 70 000, de préférence allant de 10 000 à 60 000, et mieux allant de 12 000 à 50 000.

**40.** Composition selon l'une des revendications 1 et 3 à 39, **caractérisée par le fait que** le polymère séquencé n'est pas un élastomère.

**41.** Composition selon l'une quelconque des revendications précédentes, **caractérisée par le fait que** le polymère séquence est présent en une teneur allant de 0,1 % à 60 % en poids, par rapport au' poids total de la composition, de préférence allant de 0,5 % à 50 % en poids, et de préférence encore allant de 1 % à 40 % en poids.

**42.** Composition selon l'une quelconque des revendications précédentes, **caractérisée par le fait que qu**'elle comprend un huile volatile.

**43.** Composition selon l'une quelconque des revendications précédentes, **caractérisée par le fait qu'**elle comprend un huile volatile choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane.

**44.** Composition selon la revendication 42 ou 43, **caractérisée par le fait que** l'huile volatile est présente en une teneur allant de 1 % à 70 % en poids, par rapport au poids total de la composition, de préférence allant de 5 % à 50 % en poids, et préférentiellement allant de 10 % à 35 % en poids.

**45.** Composition selon l'une quelconque des revendications précédentes, **caractérisée par le fait qu'**elle comprend une huile non volatile.

**46.** Composition selon la revendication précédente, **caractérisée par le fait que** l'huile non volatile est choisie parmi les huiles non volatiles hydrocarbonées, les huiles non volatiles siliconées.

**47.** Composition selon la revendication 45 ou 46, **caractérisée par le fait que** l'huile non volatile est présente en une teneur allant de 1 % à 80 % en poids, par rapport au poids total de la composition, de préférence allant de 5 % à 60 % en poids, et préférentiellement allant de 10 % à 50 % en poids, notamment allant de 20 % à 50 % en poids.

**48.** Composition selon l'une quelconque des revendications précédentes, **caractérisée par le fait qu'**elle comprend au moins un corps gras solides à température ambiante choisi parmi les cires, les corps gras pâteux, les gommes et leurs mélanges.

**49.** Composition selon l'une quelconque des revendications précédentes, **caractérisée par le fait qu'**elle contient de 0,1 à 50 % en poids de cires, par rapport au poids total de la composition, et de préférence de 1 à 30 % en poids.

**50.** Composition selon l'une quelconque des revendications précédentes, **caractérisée par le fait qu'**elle comprend une matière colorante.

**51.** Composition selon l'une quelconque des revendications précédentes, **caractérisée par le fait qu'**elle comprend un ingrédient cosmétique choisi parmi les polymères filmogènes additionnels, les vitamines, les épaississants, les oligo-éléments, les adoucissants, les séquestrants, les parfums, les agents alcalinisants ou acidifiants, les conservateurs, les filtres solaires, les tensioactifs, les anti-oxydants, ou leurs mélanges.

**52.** Composition cosmétique selon l'une quelconque des revendications précédentes, **caractérisée par le fait qu'**elle se présente sous forme de pâte ou de stick.

**53.** Composition cosmétique selon l'une quelconque des revendications précédentes, **caractérisée par le fait qu'**elle se présente sous forme anhydre.

**54.** Ensemble cosmétique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**55.** Ensemble cosmétique selon la revendication 54, **caractérisé par le fait que** le récipient est formé, au moins pour partie, en au moins un matériau thermoplastique.

**56.** Ensemble cosmétique selon la revendication 54, **caractérisé par le fait que** le récipient est formé, au moins pour partie, en au moins un matériau non thermoplastique, notamment en verre ou en métal.

**57.** Ensemble selon l'une quelconque des revendications 54 à 56, **caractérisé par le fait que**, en position fermée du récipient, l'élément de fermeture est vissé sur le récipient.

**58.** Ensemble selon l'une quelconque des revendications 54 à 57, **caractérisé par le fait que**, en position fermée du récipient, l'élément de fermeture est couplé au récipient autrement que par vissage, notamment par encliquetage, collage, ou soudage.

**59.** Procédé cosmétique de maquillage des lèvres comprenant l'application sur les lèvres d'une composition selon l'une quelconque des revendications 1 à 53.

**60.** Utilisation d'une composition selon l'une quelconque des revendications 1 à 53, pour obtenir un dépôt de maquillage sur les lèvres ayant une bonne tenue, notamment une bonne tenue après un contact avec de l'eau ou avec une huile alimentaire.

**Claims**

**1.** Lip makeup composition comprising at least one cosmetically acceptable organic liquid medium and at least one styrene-free film-forming linear block ethylenic polymer,
the said block polymer containing first and second blocks linked together via an intermediate segment that is a random polymer comprising at least one constituent monomer of the first block and at least one constituent monomer of the second block,
the first block of the polymer being chosen from:

- a) a block with a Tg of greater than or equal to 40°C,
- b) a block with a Tg of less than or equal to 20°C,
- c) a block with a Tg of between 20 and 40°C, and the second block being chosen from a category a), b) or c) different from the first block;

and the said block polymer having a polydispersity index I of greater than or equal to 2.8,
the said polymer being such that, when it is present in sufficient amount in the composition, the said composition is capable of forming a deposit that has a resistive index of greater than or equal to 80%, determined according to the measurement protocol described in the description.

**2.** Lip makeup composition comprising at least one cosmetically acceptable organic liquid medium and at least one non-elastomeric film-forming linear block ethylenic polymer,
the said block polymer containing first and second blocks linked together via an intermediate segment that is a random polymer comprising at least one constituent monomer of the first block and at least one constituent monomer of the second block,
the first block of the polymer being chosen from:

- a) a block with a Tg of greater than or equal to 40°C,
- b) a block with a Tg of less than or equal to 20°C,
- c) a block with a Tg of between 20 and 40°C, and the second block being chosen from a category a), b) or c) different from the first block;

and the said block polymer having a polydispersity index I of greater than or equal to 2.8,
the said polymer being such that, when it is present in sufficient amount in the composition, the said composition is capable of forming a deposit that has a resistive index of greater than or equal to 80%, determined according to the measurement protocol described in the description.

**3.** Composition according to Claim 1 or 2, **characterized in that** it is capable of forming a deposit that has a resistive index of greater than or equal to 85%, preferably less than or equal to 90%, preferably greater than or equal to 95%.

**4.** Composition according to one of the preceding claims, **characterized in that** the block polymer is not soluble at an active material content of at least 1% by weight in water or in a mixture of water and of linear or branched lower monoalcohols containing from 2 to 5 carbon atoms, without pH modification, at room temperature (25°C).

**5.** Composition according to one of the preceding claims, **characterized in that** the intermediate block has a glass transition temperature that is between the glass transition temperatures of the first and second blocks.

**6.** Composition according to any one of the preceding claims, **characterized in that** the first and second blocks of the said block polymer are mutually incompatible.

**7.** Composition according to one of the preceding claims, **characterized in that** the block polymer has a polydispersity

index of between 2.8 and 6.

8. Composition according to any one of the preceding claims, **characterized in that** the block polymer comprises at least one first block with a glass transition temperature (Tg) of greater than or equal to 40°C and at least one second block with a glass transition temperature of less than or equal to 20°C.

9. Composition according to the preceding claim, **characterized in that** the proportion of the first block ranges from 20% to 90%, better still from 30% to 80% and even better still from 50% to 70% by weight of the polymer.

10. Composition according to Claim 8 or 9, **characterized in that** the proportion of the second block with a Tg of less than or equal to 20°C ranges from 5% to 75%, better still from 15% to 50% and even better still from 25% to 45% by weight of the polymer.

11. Composition according to one of Claims 1 to 7, **characterized in that** the block polymer comprises at least one first block with a glass transition temperature (Tg) of between 20 and 40°C and at least one second block with a glass transition temperature of less than or equal to 20°C or a glass transition temperature of greater than or equal to 40°C.

12. Composition according to the preceding claim, **characterized in that** the proportion of the first block with a Tg of between 20 and 40°C ranges from 10% to 85%, better still from 30% to 80% and even better still from 50% to 70% by weight of the polymer.

13. Composition according to Claim 11 or 12, **characterized in that** the second block has a Tg of greater than or equal to 40°C.

14. Composition according to any one of Claims 11 to 13, **characterized in that** the proportion of the second block with a Tg of greater than or equal to 40°C ranges from 10% to 85%, preferably from 20% to 70% and better still from 30% to 70% by weight of the polymer.

15. Composition according to Claim 11 or 12, **characterized in that** the second block has a Tg of less than or equal to 20°C.

16. Composition according to one of Claims 7 to 9, 14 and 15, **characterized in that** the proportion of the block with a glass transition temperature of less than or equal to 20°C ranges from 20% to 90%, better still from 30% to 80% and even better still from 50% to 70% by weight of the polymer.

17. Composition according to any one of the preceding claims, **characterized in that** the block with a Tg of greater than or equal to 40°C is totally or partially derived from one or more monomers whose homopolymer has a glass transition temperature of greater than or equal to 40°C, especially a Tg ranging from 40 to 150°C, preferably greater than or equal to 50°C, especially ranging from 50°C to 120°C and preferentially greater than or equal to 60°C, especially ranging from 60°C to 120°C.

18. Composition according to the preceding claim, **characterized in that** the block with a Tg of greater than or equal to 40°C is a copolymer derived from monomers whose homopolymer has a glass transition temperature of greater than or equal to 40°C.

19. Composition according to either of Claims 17 and 18, **characterized in that** the monomers whose homopolymer has a glass transition temperature of greater than or equal to 40°C are chosen from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH\text{-}COOR_2$
in which $R_2$ represents a $C_4$ to $C_{12}$ cycloalkyl group such as isobornyl acrylate or a tert-butyl group,
- (meth)acrylamides of formula:

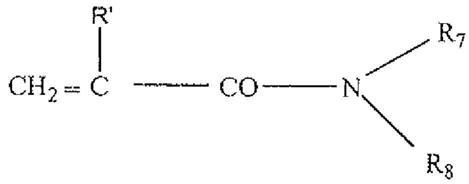

in which $R_7$ and $P_8$, which may be identical or different, each represent a hydrogen atom or a linear or branched alkyl group of 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl,
- and mixtures thereof.

**20.** Composition according to any one of Claims 17 to 19, **characterized in that** the monomers whose homopolymer has a glass transition temperature of greater than or equal to 40°C are chosen from methyl methacrylate, isobutyl methacrylate and isobornyl (meth)acrylate, and mixtures thereof.

**21.** Composition according to any one of Claims 1 to 17, 19 and 20, **characterized in that** the block with a Tg of greater than or equal to 40°C is a homopolymer.

**22.** Composition according to any one of Claims 1 to 11, 15 and 16, **characterized in that** the block with a Tg of less than or equal to 20°C is totally or partially derived from one or more monomers whose homopolymer has a glass transition temperature of less than or equal to 20°C, especially ranging from -100 to 20°C, preferably less than or equal to 15°C, especially ranging from -80°C to 15°C and preferentially less than or equal to 10°C, especially ranging from -50°C to 0°C.

**23.** Composition according to the preceding claim, **characterized in that** the monomers whose homopolymer has a glass transition temperature of less than or equal to 20°C are chosen from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms chosen from O, N and S is (are) optionally intercalated;
- methacrylates of formula $CH_2 = C(CH_3)$-$COOR_4$,
$R_4$ representing a linear or branched $C_6$ to $C_{12}$ unsubstituted alkyle group, in which one or more heteroatoms chosen from O, N and S is (are) optionally intercalated;
- vinyl esters of formula $R_5$-CO-O-CH = $CH_2$ in which $R_5$ represents a linear or branched $C_4$ to $C_{12}$ alkyl group,
- $C_4$ to $C_{12}$ alkyl vinyl ethers, such as methyl vinyl ether and ethyl vinyl ether;
- N-($C_4$ to $C_{12}$)alkyl acrylamides, such as N-octylacrylamide,
and mixtures thereof.

**24.** Composition according to Claim 22 or 23, **characterized in that** the monomers whose homopolymer has a glass transition temperature of less than or equal to 20°C are chosen from alkyl acrylates whose alkyl chain contains from 1 to 10 carbon atoms, with the exception of the tert-butyl group.

**25.** Composition according to any one of Claims 1 to 12 and 16 to 24, **characterized in that** the block with a glass transition temperature of less than or equal to 20°C is a homopolymer.

**26.** Composition according to any one of Claims 1 to 6 and 11 to 25, **characterized in that** the block with a Tg of between 20 and 40°C is totally or partially derived from one or more monomers whose homopolymer has a glass transition temperature of between 20 and 40°C.

**27.** Composition according to any one of Claims 1 to 6 and 11 to 26, **characterized in that** the block with a Tg of between 20 and 40°C is a homopolymer of a monomer chosen from n-butyl methacrylate, cyclodecyl acrylate, neopentyl acrylate and isodecylacrylamide.

**28.** Composition according to any one of Claims 1 to 6 and 11 to 26, **characterized in that** the block with a Tg of between 20 and 40°C is a copolymer totally or partially derived from:

- monomers whose homopolymer has a Tg of greater than or equal to 40°C, especially a Tg ranging from 40°C to 150°C, preferably greater than or equal to 50°C, especially ranging from 50 to 120°C, and preferentially greater than or equal to 60°C, especially ranging from 60°C to 120°C,
- and monomers whose homopolymer has a Tg of less than or equal to 20°C, especially ranging from -100 to 20°C, preferably less than or equal to 15°C, especially ranging from -80°C to 15°C, and preferentially less than or equal to 10°C, for example ranging from -50°C to 0°C.

**29.** Composition according to any one of Claims 1 to 6, 11 to 26 and 28, **characterized in that** the block with a Tg of between 20 and 40°C is totally or partially derived from monomers chosen from methyl methacrylate, isobornyl (meth)acrylate, trifluoroethyl methacrylate, butyl acrylate and 2-ethylhexyl acrylate, and mixtures thereof.

**30.** Composition according to any one of Claims 1 to 20, 22 to 24, 26, 28 and 29, **characterized in that** the first block and/or the second block comprise(s) at least one additional monomer.

**31.** Composition according to the preceding claim, **characterized in that** the additional monomer is chosen from hydrophilic monomers, monomers containing ethylenic unsaturation comprising one or more silicon atoms, and mixtures thereof.

**32.** Composition according to Claim 30 or 31, **characterized in that** the additional monomer is chosen from:

- ethylenically unsaturated monomers comprising at least one carboxylic or sulfonic acid function,
- methacrylates of formula $CH_2 = C(CH_3)\text{-}COOR_6$ in which $R_6$ represents a linear or branched alkyl group containing from 1 to 4 carbon atoms, the said alkyl group being substituted with one or more substituents chosen from hydroxyl groups and halogen atoms,
- methacrylates of formula $CH_2 = C(CH_3)\text{-}COOR_9$,
$R_9$ representing a linear or branched $C_6$ to $C_{12}$ alkyl group in which one or more heteroatoms chosen from O, N and S is (are) optionally intercalated, the said alkyl group being substituted with one or more substituents chosen from hydroxyl groups and halogen atoms;
- acrylates of formula $CH_2 = CHCOOR_{10}$,
$R_{10}$ representing a linear or branched $C_1$ to $C_{12}$ alkyl group substituted with one or more substituents chosen from hydroxyl groups and halogen atoms, or $R_{10}$ represents a $C_1$ to $C_{12}$ alkyl-O-POE (polyoxyethylene) with repetition of the oxyethylene unit 5 to 30 times, or $R_{10}$ represents a polyoxyethylenated group comprising from 5 to 30 ethylene oxide units;
- ethylenically unsaturated monomers comprising at least one tertiary amine function,
and mixtures thereof.

**33.** Composition according to any one of Claims 30 to 32, **characterized in that** the additional monomer(s) is (are) chosen from acrylic acid, methacrylic acid and trifluoroethyl methacrylate, and mixtures thereof.

**34.** Composition according to one of Claims 30 to 33, **characterized in that** the additional monomer(s) represent(s) from 1% to 30% by weight, relative to the total weight of the first and/or second blocks.

**35.** Composition according to one of Claims 1 to 33, **characterized in that** each of the first and second blocks comprises at least one monomer chosen from (meth)acrylic acid esters, and optionally at least one monomer chosen from (meth)acrylic acid, and mixtures thereof.

**36.** Composition according to one of Claims 1 to 35, **characterized in that** each of the first and second blocks is totally derived from at least one monomer chosen from (meth)acrylic acid esters, and optionally from at least one monomer chosen from (meth)acrylic acid, and mixtures thereof.

**37.** Composition according to any one of Claims 2 to 36, **characterized in that** the block polymer is styrene-free.

**38.** Composition according to one of the preceding claims, **characterized in that** the block polymer has a weight-average mass (Mw) of less than or equal to 300 000, preferably ranging from 35 000 to 200 000 and better still ranging from 45 000 to 150 000.

**39.** Composition according to one of the preceding claims, **characterized in that** the block polymer has a number-average mass (Mn) of less than or equal to 70 000, preferably ranging from 10 000 to 60 000 and better still ranging

from 12 000 to 50 000.

**40.** Composition according to one of Claims 1 and 3 to 39, **characterized in that** the block polymer is not an elastomer.

**41.** Composition according to any one of the preceding claims, **characterized in that** the block polymer is present in a content ranging from 0.1% to 60% by weight, preferably ranging from 0.5% to 50% by weight and more preferably ranging from 1% to 40% by weight, relative to the total weight of the composition.

**42.** Composition according to any one of the preceding claims, **characterized in that** it comprises a volatile oil.

**43.** Composition according to any one of the preceding claims, **characterized in that** it comprises a volatile oil chosen from octamethylcyclotetrasiloxane, decamethylcyclopentasiloxane, dodecamethylcyclohexa-siloxane, heptamethylhexyltrisiloxane, heptamethyloctyltrisiloxane, octamethyltrisiloxane, decamethyltetrasiloxane, isododecane, isodecane and isohexadecane.

**44.** Composition according to Claim 42 or 43, **characterized in that** the volatile oil is present in a content ranging from 1% to 70% by weight, preferably ranging from 5% to 50% by weight and preferentially ranging from 10% to 35% by weight, relative to the total weight of the composition.

**45.** Composition according to any one of the preceding claims, **characterized in that** it comprises a non-volatile oil.

**46.** Composition according to the preceding claim, **characterized in that** the non-volatile oil is chosen from hydrocarbon-based non-volatile oils and silicone non-volatile oils.

**47.** Composition according to Claim 45 or 46, **characterized in that** the non-volatile oil is present in a content ranging from 1% to 80% by weight, preferably ranging from 5% to 60% by weight, preferentially ranging from 10% to 50% by weight and especially ranging from 20% to 50% by weight, relative to the total weight of the composition.

**48.** Composition according to any one of the preceding claims, **characterized in that** it comprises at least one fatty substance that is solid at room temperature, chosen from waxes, pasty fatty substances and gums, and mixtures thereof.

**49.** Composition according to any one of the preceding claims, **characterized in that** it contains from 0.1% to 50% by weight and preferably from 1% to 30% by weight of waxes, relative to the total weight of the composition.

**50.** Composition according to any one of the preceding claims, **characterized in that** it comprises a dyestuff.

**51.** Composition according to any one of the preceding claims, **characterized in that** it comprises a cosmetic ingredient chosen from additional film-forming polymers, vitamins, thickeners, trace elements, softeners, sequestrants, fragrances, acidifying or basifying agents, preserving agents, sunscreens, surfactants and antioxidants, or mixtures thereof.

**52.** Cosmetic composition according to any one of the preceding claims, **characterized in that** it is in the form of a paste or a stick.

**53.** Cosmetic composition according to any one of the preceding claims, **characterized in that** it is in anhydrous form.

**54.** Cosmetic assembly comprising:

a) a container delimiting at least one compartment, the said container being closed by a closing member; and
b) a composition placed inside the said compartment, the composition being in accordance with any one of the preceding claims.

**55.** Cosmetic assembly according to Claim 54, **characterized in that** the container is at least partially formed from at least one thermoplastic material.

**56.** Cosmetic assembly according to Claim 54, **characterized in that** the container is at least partially formed from at least one non-thermoplastic material, especially glass or metal.

**57.** Assembly according to any one of Claims 54 to 56, **characterized in that**, in the closed position of the container, the closing member is screwed onto the container.

**58.** Assembly according to any one of Claims 54 to 57, **characterized in that**, in the closed position of the container, the closing member is coupled to the container other than by screwing, especially by click-fastening, bonding or welding.

**59.** Cosmetic process for making up the lips, comprising the application to the lips of a composition according to any one of Claims 1 to 53.

**60.** Use of a composition according to any one of Claims 1 to 53, to obtain a deposit of makeup on the lips that has good resistance, in particular good resistance after coming into contact with water or with a dietary oil.

## Patentansprüche

**1.** Zusammensetzung zum Schminken der Lippen, die mindestens ein kosmetisch akzeptables, flüssiges organisches Medium und mindestens ein filmbildendes, lineares, ethylenisches Sequenzpolymer frei von Styrol enthält,
wobei das Sequenzpolymer eine erste Sequenz und eine zweite Sequenz enthält, die über ein Zwischensegment aneinander gebunden sind, bei dem es sich um ein statistisches Polymer handelt, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I von größer oder gleich 2,8 aufweist,
und wobei das Polymer so ist, dass die Zusammensetzung, wenn es in der Zusammensetzung in einer ausreichenden Menge enthalten ist, ein Depot bilden kann, dessen gemäß der in der Beschreibung angegebenen Vorgehensweise ermittelte Festigkeitsindex größer oder gleich 80 % ist.

**2.** Zusammensetzung zum Schminken der Lippen, die mindestens ein kosmetisch akzeptables, flüssiges organisches Medium und mindestens ein nicht elastomeres, filmbildendes, lineares, ethylenisches Sequenzpolymer enthält,
wobei das Sequenzpolymer eine erste Sequenz und eine zweite Sequenz enthält, die über ein Zwischensegment aneinander gebunden sind, bei dem es sich um ein statistisches Polymer handelt, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40°C;

und die zweite Sequenz aus einer von der ersten Sequenz verschiedenen Gruppe a), b) oder c) ausgewählt ist;
wobei das Sequenzpolymer einen Polydispersitätsindex 1 von größer oder gleich 2,8 aufweist,
und wobei das Polymer so ist, dass die Zusammensetzung, wenn es in der Zusammensetzung in einer ausreichenden Menge enthalten ist, ein Depot bilden kann, dessen gemäß der in der Beschreibung angegebenen Vorgehensweise ermittelte Festigkeitsindex größer oder gleich 80 % ist.

**3.** Zusammensetzung nach Anspruch 1 oder 2, **dadurch gekennzeichnet, dass** sie befähigt ist, ein Depot zu bilden, dessen Festigkeitsindex größer oder gleich 85 %, vorzugsweise kleiner oder gleich 90 % und noch bevorzugter größer oder gleich 95 % ist.

**4.** Zusammensetzung nach einem der vorhergehenden Ansprüche, **dadurch gekennzeichnet, dass** das Polymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25 °C) nicht löslich ist.

5. Zusammensetzung nach dem vorhergehenden Anspruch,
**dadurch gekennzeichnet, dass** die Zwischensequenz eine Glasübergangstemperatur aufweist, die zwischen den Glasübergangstemperaturen der ersten und der zweiten Sequenz liegt.

6. Zusammensetzung nach einem der vorhergehenden Ansprüche, **dadurch gekennzeichnet, dass** die erste Sequenz und die zweite Sequenz des Sequenzpolymers nicht miteinander kompatibel sind.

7. Zusammensetzung nach einem der vorhergehenden Ansprüche, **dadurch gekennzeichnet, dass** das Sequenzpolymer einen Polydispersitätsindex im Bereich von 2,8 bis 6 aufweist.

8. Zusammensetzung nach einem der vorhergehenden Ansprüche, **dadurch gekennzeichnet, dass** das Sequenzpolymer zumindest eine erste Sequenz mit einer Glasübergangstemperatur (Tg) von größer oder gleich 40 °C und zumindest eine zweite Sequenz mit einer Glasübergangstemperatur von kleiner oder gleich 20 °C umfasst.

9. Zusammensetzung nach dem vorhergehenden Anspruch,
**dadurch gekennzeichnet, dass** der Anteil der ersten Sequenz im Bereich von 20 bis 90 Gew.-% des Polymers, besser im Bereich von 30 bis 80 Gew.-% und besser im Bereich von 50 bis 70 Gew.-% liegt.

10. Zusammensetzung nach Anspruch 8 oder 9, **dadurch gekennzeichnet, dass** der Anteil der zweiten Sequenz mit einer Tg von kleiner oder gleich 20 °C im Bereich von 5 bis 75 Gew.-% des Polymers, besser im Bereich von 15 bis 50 Gew.-% und noch besser im Bereich von 25 bis 45 Gew.-% liegt.

11. Zusammensetzung nach einem der Ansprüche 1 bis 7, **dadurch gekennzeichnet, dass** das Sequenzpolymer mindestens eine erste Sequenz mit einer Glasübergangstemperatur (Tg) zwischen 20 und 40 °C und zumindest eine zweite Sequenz mit einer Glasübergangstemperatur von kleiner oder gleich 20 °C oder einer Glasübergangstemperatur von größer oder gleich 40 °C umfasst.

12. Zusammensetzung nach dem vorhergehenden Anspruch,
**dadurch gekennzeichnet, dass** der Anteil der ersten Sequenz mit einer Tg zwischen 20 und 40 °C im Bereich von 10 bis 85 Gew.-% des Polymers, besser im Bereich von 30 bis 80 Gew.-% und noch besser im Bereich von 50 bis 70 Gew.-% liegt.

13. Zusammensetzung nach Anspruch 11 oder 12, **dadurch gekennzeichnet, dass** die zweite Sequenz eine Tg von größer oder gleich 40 °C aufweist.

14. Zusammensetzung nach einem der Ansprüche 11 bis 13,
**dadurch gekennzeichnet, dass** der Anteil der zweiten Sequenz mit einer Tg von größer oder gleich 40 °C im Bereich von 10 bis 85 Gew.-%, vorzugsweise im Bereich von 20 bis 70 Gew.-% und besser im Bereich von 30 bis 70 Gew.-% des Polymers liegt.

15. Zusammensetzung nach Anspruch 11 oder 12, **dadurch gekennzeichnet, dass** die zweite Sequenz eine Tg von kleiner oder gleich 20 °C aufweist.

16. Zusammensetzung nach einem der Ansprüche 7 bis 9 und 14, 15, **dadurch gekennzeichnet, dass** der Anteil der Sequenz mit einer Glasübergangstemperatur von kleiner oder gleich 20°C im Bereich von 20 bis 90 Gew.-% des Polymers, besser im Bereich von 30 bis 80 Gew.-% und noch besser im Bereich von 50 bis 70 Gew.-% liegt.

17. Zusammensetzung nach einem der vorhergehenden Ansprüche, **dadurch gekennzeichnet, dass** die Sequenz, die eine Tg von 40 °C oder darüber aufweist, ganz oder teilweise von einem oder mehreren Monomeren abgeleitet ist, deren Homopolymer eine Glasübergangstemperatur von größer oder gleich 40°C, insbesondere eine Tg im Bereich von 40 bis 150 °C, vorzugsweise von 50 °C oder darüber, insbesondere im Bereich von 50 bis 120 °C und bevorzugt von 60 °C oder darüber, insbesondere im Bereich von 60 bis 120 °C aufweist.

18. Zusammensetzung nach dem vorhergehenden Anspruch,
**dadurch gekennzeichnet, dass** die Sequenz, die eine Tg von 40 °C oder darüber aufweist, ein Copolymer ist, das von Monomeren abgeleitet ist, deren Homopolymer eine Glasübergangstemperatur von 40 °C oder darüber aufweist.

**19.** Zusammensetzung nach einem der Ansprüche 17 oder 18,
**dadurch gekennzeichnet, dass** die Monomere, deren Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$, worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen ist,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

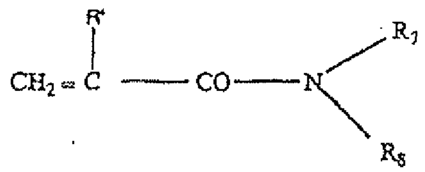

worin $R_7$ und $R_8$, die gleich oder verschieden sind, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet; und deren Gemischen.

**20.** Zusammensetzung nach einem der Ansprüche 17 bis 19, **dadurch gekennzeichnet, dass** die Monomere, deren Homopolymer eine Glasübergangstemperatur von größer oder gleich 40 °C aufweist, unter Methylmethacrylat, Isobutylrnethacrylat und Isobornyl(meth)acrylat und deren Gemischen ausgewählt sind.

**21.** Zusammensetzung nach einem der Ansprüche 1 bis 17 und 19, 20, **dadurch gekennzeichnet, dass** die Sequenz, die eine Tg von 40 °C oder darüber aufweist, ein Homopolymer ist.

**22.** Zusammensetzung nach einem der Ansprüche 1 bis 11 und 15, 16, **dadurch gekennzeichnet, dass** die Sequenz, die eine Tg von 20 °C oder darunter aufweist, ganz oder teilweise von einem oder mehreren Monomeren abgeleitet ist, deren Homopolymer eine Glasübergangstemperatur von 20 °C oder darunter, insbesondere im Bereich von -100 bis 20 °C, vorzugsweise von 15 °C oder darunter, insbesondere im Bereich von -80 bis 15 °C und bevorzugt von 10 °C oder darunter, insbesondere im Bereich von -50 bis 0 °C aufweist.

**23.** Zusammensetzung nach dem vorhergehenden Anspruch,
**dadurch gekennzeichnet, dass** die Monomere, deren Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind);
- Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind);
- Vinylestern der Formel $R_5-CO-O-CH=CH_2$,
worin $R_5$ eine lineare oder verzweigte Alkylgruppe mit 4 bis 12 Kohlenstoffatomen bedeutet;
- Alkyl($C_{4-12}$)vinylethern, wie Methylvinylether und Ethylvinylether;
- N-Alkyl($C_4$-$_{12}$)acrylamiden, wie N-Octylacrylamid,
- und deren Gemischen.

**24.** Zusammensetzung nach Anspruch 22 oder 23, **dadurch gekennzeichnet, dass** die Monomere, deren Homopolymer eine Glasübergangstemperatur von 20 °C oder darunter aufweist, unter den Alkylacrylaten ausgewählt ist, deren Alkylkette 1 bis 10 Kohlenstoffatome aufweist, wobei die Gruppe tert-Butyl ausgenommen ist.

**25.** Zusammensetzung nach einem der Ansprüche 1 bis 12 und 16 bis 24, **dadurch gekennzeichnet, dass** die Sequenz mit einer Glasübergangstemperatur von 20 °C oder darunter ein Homopolymer ist.

**26.** Zusammensetzung nach einem der Ansprüche 1 bis 6 und 11 bis 25, **dadurch gekennzeichnet, dass** die Sequenz mit einer Tg zwischen 20 und 40 °C ganz oder veilweise von einem oder mehreren Monomeren stammt, deren Homopolymer eine Glasübergangstemperatur zwischen 20 und 40 °C aufweist.

**27.** Zusammensetzung nach einem der Ansprüche 1 bis 6 und 11 bis 26, **dadurch gekennzeichnet, dass** die Sequenz mit einer Tg zwischen 20 und 40°C ein Homopolymer eines Monomers ist, das unter n-Butylmethacrylat, Cyclodecylacrylat, Neopentylacrylat und Isodecylacrylamid ausgewählt ist.

**28.** Zusammensetzung nach einem der Ansprüche 1 bis 6 und 11 bis 26, **dadurch gekennzeichnet, dass** die Sequenz mit einer Tg zwischen 20 und 40 °C ein Copolymer ist, das ganz oder teilweise abgeleitet ist von:

- Monomeren, deren Homopolymer eine Tg von größer oder gleich 40 °C, insbesondere eine Tg im Bereich von 40 bis 150 °C, vorzugsweise von größer oder gleich 50 °C, insbesondere im Bereich von 50 bis 120 °C und bevorzugt von größer oder gleich 60 °C und insbesondere im Bereich von 60 bis 120 °C aufweist; und
- Monomeren, deren Homopolymer eine Tg von kleiner oder gleich 20 °C, insbesondere im Bereich von -100 bis 20 °C, vorzugsweise von 15 °C oder darunter, insbesondere im Bereich von -80 bis 15 °C und bevorzugt von 10 °C oder darunter, beispielsweise im Bereich von -50 bis 0 °C aufweist.

**29.** Zusammensetzung nach einem der Ansprüche 1 bis 6 und 11 bis 26 und 28, **dadurch gekennzeichnet, dass** die Sequenz mit einer Tg zwischen 20 und 40°C ganz oder teilweise von Monomeren abgeleitet ist, die unter Methylmethacrylat, Isobornyl(meth)acrylat, Trifluorethylmethacrylat, Butylacrylat, 2-Ethylhexylacrylat und deren Gemischen ausgewählt sind.

**30.** Zusammensetzung nach einem der Ansprüche 1 bis 20 und 22 bis 24 und 26, 28, 29, **dadurch gekennzeichnet, dass** die erste Sequenz und/oder die zweite Sequenz mindestens ein ergänzendes Monomer enthalten.

**31.** Zusammensetzung nach dem vorhergehenden Anspruch,
**dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**32.** Zusammensetzung nach Anspruch 30 oder 31, **dadurch gekennzeichnet, dass** das ergänzende Monomer ausgewählt ist unter:

- Monomeren mit einer oder mehreren ethylenisch ungesättigten Bindungen, die mindestens eine Carbonsäurefunktion oder Sulfonsäurefunktion enthalten,
- Methacrylaten der Formel $CH_2 = C(CH_3)\text{-}COOR_6$, wobei $R_6$ eine geradkettige oder verzweigte Alkylgruppe mit 1 bis 4 Kohlenstoffatomen bedeutet, wobei die Alkylgruppe mit einem oder mehreren Substituenten substituiert ist, die unter der Hydroxygruppe und den Halogenatomen ausgewählt sind;
- Methacrylaten der Formel $CH_2 = C(CH_3)\text{-}COOR_9$, wobei $R_9$ eine geradkettige oder verzweigte $C_{6\text{-}12}$-Alkylgruppe bedeutet, in deren Kette gegebenenfalls ein oder mehrere Heteroatome eingebaut ist (sind), die unter O, N und S ausgewählt sind, wobei die Alkylgruppe mit einem oder mehreren Substituenten substituiert ist, die unter der Hydroxygruppe und den Halogenatomen ausgewählt sind;
- Acrylaten der Formel $CH_2 = CHCOOR_{10}$,
wobei $R_{10}$ eine geradkettige oder verzweigte $C_{1\text{-}12}$-Alkylgruppe bedeutet, die mit einem oder mehreren Substituenten substituiert ist, die unter der Hydroxygruppe und den Halogenatomen ausgewählt sind, oder $R_{10}$ Alkyl $(C_{1\text{-}12})$-O-POE (Polyoxyethylen) mit 5 bis 30 Oxyethylen-Wiederholungseinheiten bedeutet, oder $R_{10}$ eine Polyoxyethylengruppe mit 5 bis 30 Ethylenoxideinheiten ist,
- Monomeren mit einer oder mehreren ethylenisch ungesättigten Bindungen, die mindestens eine tertiäre Aminofunktion enthalten,
- und deren Gemischen.

**33.** Zusammensetzung nach einem der Ansprüche 30 bis 32,
**dadurch gekennzeichnet, dass** das oder die ergänzenden Monomere unter Acrylsäure, Methacrylsäure, Trifluorethylmethacrylat und deren Gemischen ausgewählt ist.

**34.** Zusammensetzung nach einem der Ansprüche 30 bis 33,
**dadurch gekennzeichnet, dass** das oder die ergänzende(n) Monomer(e) 1 bis 30 Gew.-% des Gesamtgewichts der ersten und/oder zweiten Sequenz ausmachen.

**35.** Zusammensetzung nach einem der Ansprüche 1 bis 33,
**dadurch gekennzeichnet, dass** die erste und die zweite Sequenz jede mindestens ein Monomer enthalten, das unter den Estern der (Meth)acrylsäure ausgewählt ist, und gegebenenfalls mindestens ein Monomer, das unter (Meth)acrylsäure ausgewählt ist, und deren Gemische.

**36.** Zusammensetzung nach einem Ansprüche 1 bis 35, **dadurch gekennzeichnet, dass** die erste und die zweite Sequenz jede vollständig von mindestens einem Monomer, das unter den Estern der (Meth)acrylsäure ausgewählt ist, und gegebenenfalls mindestens einem Monomer, das unter (Meth)acrylsäure ausgewählt ist, und deren Gemischen abgeleitet ist.

**37.** Zusammensetzung nach einem der Ansprüche 2 bis 38,
**dadurch gekennzeichnet, dass** das Sequenzpolymer kein Styrol aufweist.

**38.** Zusammensetzung nach einem der vorhergehenden Ansprüche, **dadurch gekennzeichnet, dass** das Sequenzpolymer eine gewichtsmittlere Molmasse (Mw) von kleiner oder gleich 300 000, vorzugsweise im Bereich von 35 000 bis 200 000 und noch besser im Bereich von 45 000 bis 150 000 aufweist.

**39.** Zusammensetzung nach einem der vorhergehenden Ansprüche, **dadurch gekennzeichnet, dass** das Sequenzpolymer eine zahlenmittlere Molmasse (Mn) aufweist, die kleiner oder gleich 70 000 ist und vorzugsweise im Bereich von 10 000 bis 60 000 und noch besser im Bereich von 12 000 bis 50 000 liegt.

**40.** Zusammensetzung nach einem der Ansprüche 1 und 3 bis 39, **dadurch gekennzeichnet, dass** das Sequenzpolymer kein Elastomer ist.

**41.** Zusammensetzung nach einem der vorhergehenden Ansprüche, **dadurch gekennzeichnet, dass** das Sequenzpolymer in einem Mengenanteil von 0,1 bis 60 Gew.-%, bezogen auf das Gesamtgewicht der Zusammensetzung, vorzugsweise 0,5 bis 50 Ges.-% und noch bevorzugter 1 bis 40 Gew.-% enthalten ist.

**42.** Zusammensetzung nach einem der vorhergehenden Ansprüche, **dadurch gekennzeichnet, dass** sie ein flüchtiges Öl enthält.

**43.** Zusammensetzung nach einem der vorhergehenden Ansprüche, **dadurch gekennzeichnet, dass** sie ein flüchtiges Öl enthält, das unter Octamethylcyclotetrasiloxan, Decamethylcyclopentasiloxan, Dodecamethylcyclohexasiloxan, Heptamethylhexyltrisiloxan, Heptamethyloctyltrisiloxan, Octamethyltrisiloxan, Decamethyltetrasiloxan, Isododecan, Isodecan und Isohexadecan ausgewählt ist.

**44.** Zusammensetzung nach Anspruch 42 oder 43, **dadurch gekennzeichnet, dass** das flüchtige Öl in einem Mengenanteil von 1 bis 70 Gew.-%, bezogen auf das Gesamtgewicht der Zusammensetzung, vorzugsweise im Bereich von 5 bis 50 Gew.-% und noch bevorzugter im Bereich von 10 bis 35 Gew.-% enthalten ist.

**45.** Zusammensetzung nach einem der vorhergehenden Ansprüche, **dadurch gekennzeichnet, dass** sie ein nicht flüchtiges Öl enthält.

**46.** Zusammensetzung nach dem vorhergehenden Anspruch,
**dadurch gekennzeichnet, dass** das nicht flüchtige Öl unter den nicht flüchtigen Kohlenwasserstoffölen und den nicht flüchtigen Siliconölen ausgewählt ist.

**47.** Zusammensetzung nach Anspruch 45 oder 46, **dadurch gekennzeichnet, dass** das nicht flüchtige Öl in einem Mengenanteil von 1 bis 80 Gew.-%, bezogen auf das Gesamtgewicht der Zusammensetzung, vorzugsweise im Bereich von 5 bis 60 Gew.-%, noch bevorzugter im Bereich von 10 bis 50 Gew.-% und insbesondere im Bereich von 20 bis 50 Ges.-% enthalten ist.

**48.** Zusammensetzung nach einem der vorhergehenden Ansprüche, **dadurch gekennzeichnet, dass** sie mindestens eine bei Raumtemperatur feste Fettsubstanz enthält, die unter den Wachsen, pastösen Fettsubstanzen, Gummis

und deren Gemischen ausgewählt ist.

**49.** Zusammensetzung nach einem der vorhergehenden Ansprüche, **dadurch gekennzeichnet, dass** sie 0,1 bis 50 Gew.-% Wachse, bezogen auf das Gesamtgewicht der Zusammensetzung, und vorzugsweise 1 bis 30 Gew.-% enthält.

**50.** Zusammensetzung nach einem der vorhergehenden Ansprüche, **dadurch gekennzeichnet, dass** sie ein Farbmittel enthält.

**51.** Zusammensetzung nach einem der vorhergehenden Ansprüche, **dadurch gekennzeichnet, dass** sie einen kosmetischen Bestandteil enthält, der unter den ergänzenden filmbildenden Polymeren, Vitaminen, Verdickungsmitteln, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien oder deren Gemischen ausgewählt ist.

**52.** Kosmetische Zusammensetzung nach einem der vorhergehenden Ansprüche, **dadurch gekennzeichnet, dass** sie als Paste oder Stift vorliegt.

**53.** Kosmetische Zusammensetzung nach einem der vorhergehenden Ansprüche, **dadurch gekennzeichnet, dass** sie in wasserfreier Form vorliegt.

**54.** Kosmetische Einheit umfassen:

a) einen Behälter, der mindestens eine Abteilung abgrenzt, wobei der Behälter mit einer Verschlusseinrichtung verschlossen ist, und
b) eine Zusammensetzung, die sich im Inneren der Abteilung befindet, wobei die Zusammensetzung einer Zusammensetzung nach einem der vorhergehenden Ansprüche entspricht.

**55.** Kosmetische Einheit nach Anspruch 54, **dadurch gekennzeichnet, dass** der Behälter zumindest zum Teil aus mindestens einem thermoplastischen Material gebildet ist.

**56.** Kosmetische Einheit nach Anspruch 54, **dadurch gekennzeichnet, dass** der Behälter zumindest zum Teil aus mindestens einem nichtthermoplastischen Material, insbesondere aus Glas oder Metall, gebildet ist.

**57.** Einheit nach einem der Ansprüche 54 bis 56, **dadurch gekennzeichnet, dass** in der geschlossenen Stellung des Behälters die Verschlusseinrichtung mit dem Behälter verschraubt ist.

**58.** Einheit nach einem der Ansprüche 54 bis 57, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**59.** Kosmetisches Verfahren zum Schminken der Lippen, das das Auftragen einer Zusammensetzung nach einem der Ansprüche 1 bis 53 auf die Lippen umfasst.

**60.** Verwendung einer Zusammensetzung nach einem der Ansprüche 1 bis 53, um auf den Lippen ein Depot der Schminke mit hoher Festigkeit zu bilden, insbesondere hoher Festigkeit nach Kontakt mit Wasser oder einem Speiseöl.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6153206 A
- FR 2809306
- FR 2832719
- FR 2809306 A
- WO 03046032 A
- FR 2722380
- US 5492426 A
- FR 2761959
- WO 0103538 A
- FR 2806273
- FR 2775566
- FR 2727609
- WO 03018423 A
- FR 2791042
- FR 2792618


### Littérature non-brevet citée dans la description


- Polymer Handbook. John Wiley, 1989